# EUROPEAN PATENT APPLICATION

(11) **EP 4 049 634 A1**
(43) Date of publication of application: **31.08.2022**
(21) Application number: 20879735.7
(22) Date of filing: 22.10.2020
(51) Int. Cl.: A61F 5/00

(54) **ENDOSCOPIC GASTROINTESTINAL DEVICE FOR RESTRICTION AND REDUCING FOOD ABSORPTION WITH A POSITIONING SYSTEM IN THE STOMACH AND EXTENSION TO THE INTESTINE**

(30) Priority: 25.10.2019 BR 102019022526
(71) Applicant: Cairo Nunes, Gabriel, 14804-475 Araraquara - SP (BR)
(72) Inventor: Cairo Nunes, Gabriel, 14804-475 Araraquara - SP (BR)
(74) Representative: Pereira da Cruz, Joao
(86) International application number: PCT/BR2020/050431
(87) International publication number: WO 2021/077189

(57) **Abstract**

The invention relates to the absorption reduction technique, inhibiting hunger and reducing the quantity of food ingested, and also preventing contact between food and a portion of the intestine, with a view to reducing patient weight and related diseases, such as type 2 diabetes. For this purpose, a gastrointestinal device (10) comprises an intragastric ring (1) made of flexible and expandable material extending a tubular sleeve (3) that may be fitted with a stent. The inlet valve (4) of the gastrointestinal device (10) is coupled to a catheter (CA) for the gastrointestinal implant, being guided inwards through the stomach (E) of the patient, where endoscopic forceps (PE) or the stent guides the tubular sleeve (3) along the pyloric channel (P), entering the duodenum (D) and unfolding to occupy a portion of the intestine. The intragastric ring (1) is thus positioned beside the pyloric channel (P) inside the stomach (E) before being inflated and expanded by fluid-liquid, gas or air to assume the shape of a balloon.

## Description

### Technical field

This descriptive report relates to an invention patent application for a novel combination of an Intragastric ring communicating with an intestinal sleeve, with or without stent, and coupled to the Intragastric ring.

This gastrointestinal device is guided deflated through the stomach of the patient by means of a stomach endoscopy procedure. The Intragastric ring is positioned above the pyloric orifice and, after fluid inflation - either liquid, air or gas, from its inlet valve (by the catheter that leads it), assuming a spherical shape free of tips or corners, occupying part of the stomach and anchoring the tubular sleeve, unwound in the duodenum and a portion of the intestine. The unwinding and positioning of the sleeve may be performed by endoscopic forceps or stent (if any).

With this structure, even with reduced dimensions the Intragastric ring does not migrate to the intestine due to the size it reaches when inflated and due to the retention caused by the anchoring of the intestinal sleeve itself, remaining stable without the need for clamps or tethers and being minimally exposed in the stomach.

Thus, part of the food processed by the stomach (with its completely free walls) passes through the hollow part of the intragastric ring, having its absorption flow decreased by following through the intestine lined by the tubular intestinal sleeve.

In this way, the gastrointestinal device, occupying part of the stomach, acts in a restrictive manner, inhibiting hunger and reducing the food intake. In this absorption reduction technique, the sleeve prevents the contact between food and a portion of the intestine, thus reducing absorption, helping the patient with losing weight and fighting related diseases such as type 2 diabetes.

### Comments on the state of the art

As known, not only by the medical community but also by society in general, overweight and obesity are present in more than 50% of the world population. In developing countries like Brazil, these rates include to more than 60% of the population. Overweight and obesity are inherently associated to a large number of diseases, the so-called comorbidities, among which are diabetes, hypertension, sleep apnea, osteoarticular diseases and hepatic steatosis, some types of cancer and many other diseases. The amount of extra weight is directly related to the increase in the worldwide death toll. Most treatments intended to combat this scourge are fleeting and therefore the indication for bariatric surgery has been growing.

However, there are currently intragastric devices that are entered into the patient's stomach via transoral procedure, which means through the oral channel, and that are then expanded by fluid under the action of a valve.

In this context, the patent document BR 11 2019 006079 0 can be cited, which has two images inserted by way of example, as Figure 1, for the patent application to be described later. As can be observed from these images, the device has a tubular body (2) provided with stents or meshes (202), surrounded by a balloon structure (3) and having a terminal ring (4), from which extends a duodenojejunal by-pass sleeve (101). The tubular body (2) is taken by endoscopic procedure to the stomach with the balloon (3) deflated, having the terminal ring (4) positioned below the pylorus. After inflated, the balloon (3), by its parts (31) and (32), coats the stomach walls and its duodenojejunal by-pass sleeve (101) is extended, as lining, along the patient's intestine.

Another patent document, BR 11 2015 000384 2, is cited which has two images inserted also by way of example, as Figure 2, for the patent application to be described later. As can be seen from these two images, the device comprises a ring (220) with a tether (224) for attachment to a sleeve (202) with a corrugated edge (2810) elastic wire, or with an edge surrounded by a stent (422). As the device is transported, folded, through the interior of the gastrointestinal tract, by the elastic corrugation (2810) or by the stent (422), the sleeve (202) is attached below the Pylorus and extends into the interior of the intestine, thus lining the duodenal wall and keeping the ring (220), connected by the tether (224), positioned in the patient's stomach. As observed, the ring (220) is secured by means of welds (228) to the patient's stomach wall.

The aforementioned device is part of a method called "Endobarrier", whose images, extracted from Google, are inserted also by way of example, as Figure 3, for the patent application to be described later. It is a method developed for non-surgical treatment in patients with type 2 diabetes and obesity, still as an experimental approach. As shown in these images, the device is a body of impermeable polytetrafluoroethylene membrane that extends through the duodenum, being carried endoscopically with the patient under anaesthesia. Its edge is wave-shaped cut and clips with fastening tethers are passed just below the pyloric orifice. The Endobarrier device is transported through the patient's stomach via the normal operating channel of a standard gastroscope, by probe, receiving the filling fluid through its jagged edge, and inflating the membrane to initiate treatment.

Therefore, known devices (gastrointestinal implants), such as those disclosed above, may bring discomfort to the patient's gastrointestinal tract during treatment. There is a concern to avoid occlusion, migration of the intragastric element, and also other factors that may impair the treatment, such as the undesirable seal (marked contact of an inflated balloon with the stomach walls (Fig. 1)), or which may require anchor points, through clips, clamps and tethers susceptible of perforating the walls of the stomach or intestine (Figs. 2 and 3).

### Objectives of the invention

The inflatable gastrointestinal endoscopic device, which is the object of this patent application, due to its constructiveness and the system surrounding it, proposes the positioning of an Intragastric ring immediately above the pyloric orifice, keeping it stabilized and providing for the set-up of an intestinal sleeve in the duodenum and part of the intestine, preventing perforations from occurring and leaving the stomach walls (which naturally suffer contractions) completely free to process food.

For this purpose, a valve is installed in the body of the Intragastric ring, which extends a tubular sleeve, provided or not with a (spiral or rectilinear) stent. By means of the proposed positioning system, through its valve connected to a catheter, this set of Intragastric ring coupled with the intestinal sleeve is led entirely folded to the Pylorus. After positioning the Intragastric ring above the entrance of the pyloric channel, the ring is inflated with air, gas or liquid, and then the intestinal sleeve is extended along the duodenum and part of the intestine, with the aid of endoscopic forceps or stent. In this condition, the Intragastric ring, inflated like a balloon, is stabilized above the entrance to the pyloric channel without being attached and the intestinal sleeve remains distended along the walls of the duodenum and part of the intestine.

Therefore, without the use of any type of clip, clamp, binding or coating on the stomach walls, the Intragastric ring thus stabilized allows the passage of part of the processed food, and the passage of this part of processed food through the intestinal sleeve along the duodenum and a portion of the intestine, thus reducing the absorption of the ingested food content and reducing the absorption of food by the intestine, resulting in a treatment against obesity and diseases associated to overweight.

The gastrointestinal device is thus kept acting in a restrictive and disabsorptive manner (as a gastric bypass surgery), but without the surgical risk, also being reversible or reapplicable.

### Brief description of Figures

Having been broadly explained, the invention is hereinafter detailed.

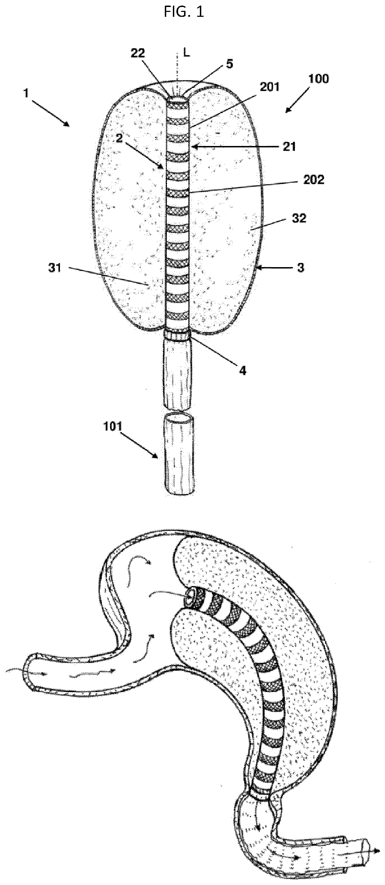

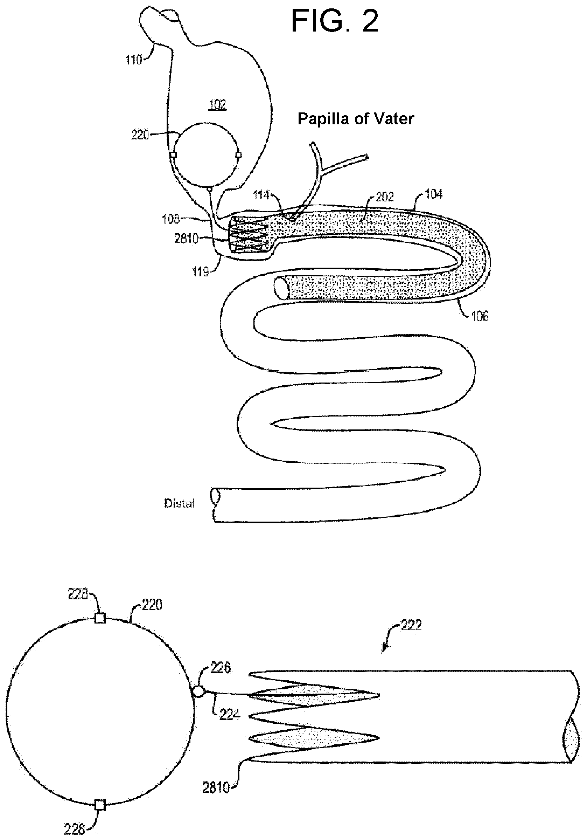

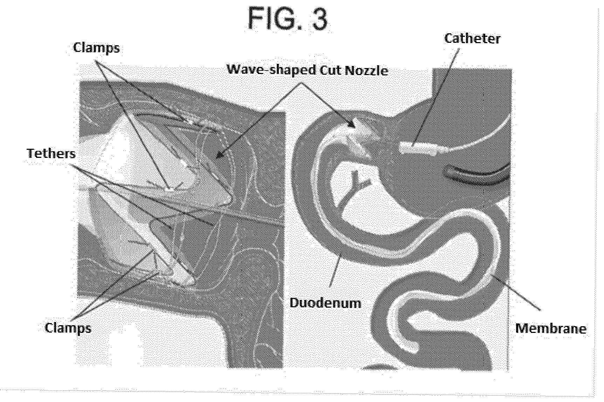

Figures 1, 2 and 3, as already mentioned, refer to the prior art, shown by the patent documents BR 11 2019 006079 0, BR 11 2015 000384 2, and by the images of the "Endo barrier" device.

The following figures, from 4 to 14, show the Intragastric ring coupled to the intestinal sleeve and its positioning system, object of this patent application, to be further detailed through the figures listed below:

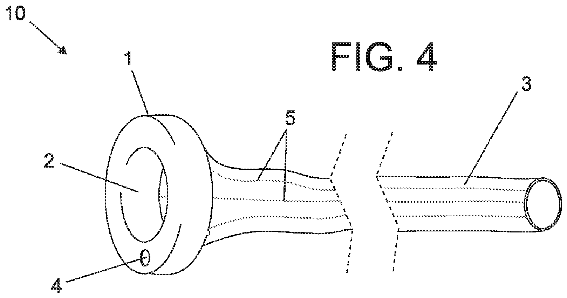

Figure 4 - illustrates the gastrointestinal device with the corrugated tubular intestinal sleeve;

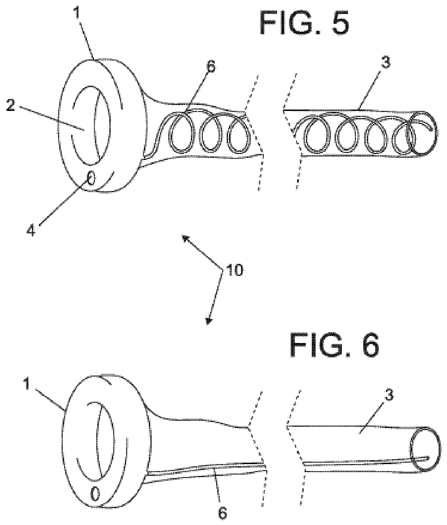

Figure 5 - illustrates the gastrointestinal device in a configuration where the tubular sleeve adopts a spiral stent;
Figure 6 - illustrates the gastrointestinal device in a configuration where the tubular sleeve adopts a rectilinear stent;

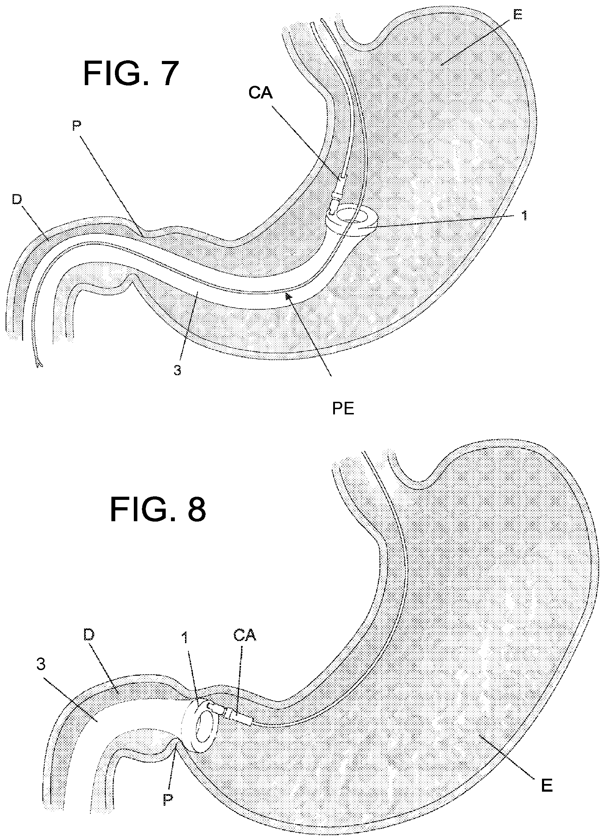

Figure 7 - illustrates the gastrointestinal device being guided inwards through the stomach, aided by the catheter, in an endoscopy procedure;
Figure 8 - view of the Intragastric ring of the device already positioned above the pyloric channel, the intestinal sleeve being extended through the duodenum and a portion of the intestine with the use of endoscopic forceps;

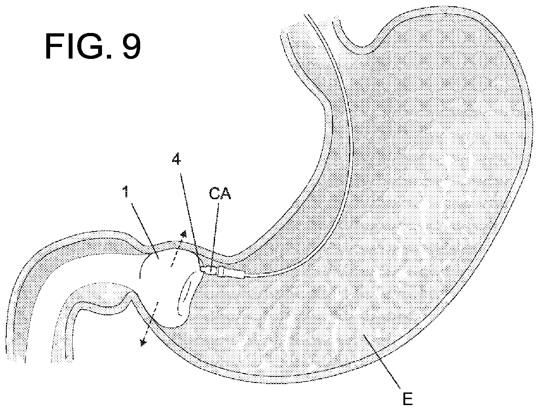

Figure 9 - illustrates the positioned device, the fluid (liquid, air or gas) being injected to fill the ring, from the catheter and through the valve, when the Intragastric ring is inflated like a balloon until it acquires the shape of the lower stomach walls. With this, the Intragastric ring is kept in a stabilized position above the pyloric channel and minimally exposed in the stomach;

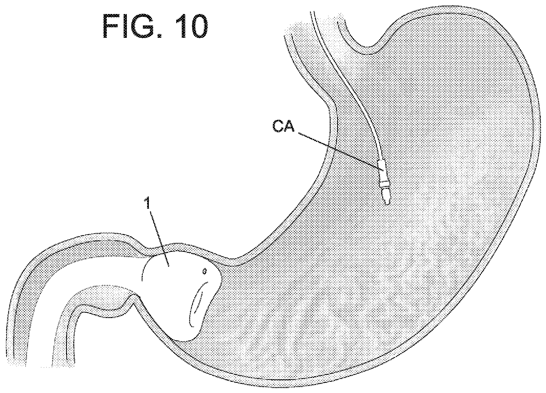

Figure 10 - illustrates the above positioning, with the ring inflated, wherein the catheter is removed and the device is ready to perform its functions;

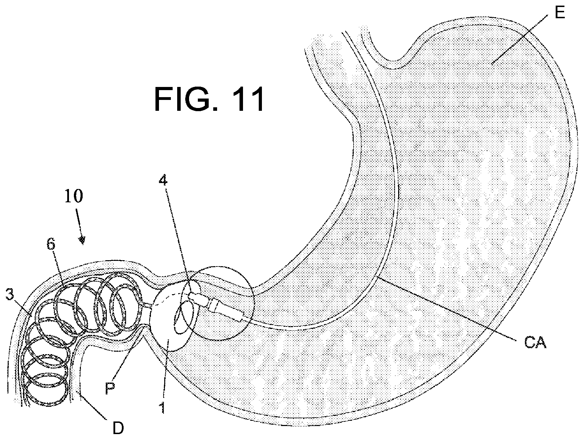

Figure 11 - illustrates the positioning system shown in the previous figures, but with the tubular sleeve fitted with a stent, which is inflated together with the ring, unwinding the tubular sleeve through the duodenum and part of the intestine without the need for endoscopic forceps;

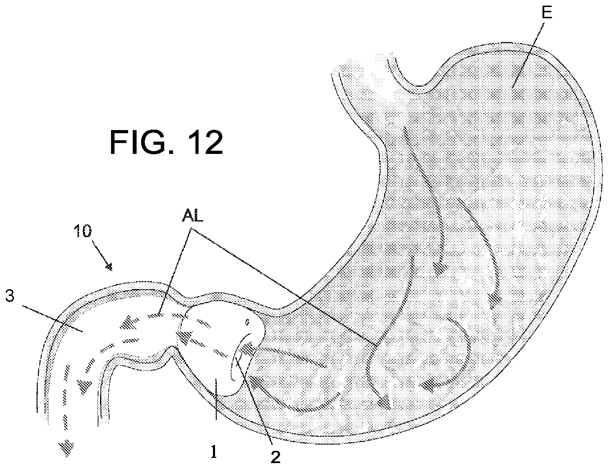

Figure 12 - illustrates the device positioned on the patient. The latter, when eating, has the processed food directed by the central nozzle of the inflated ring and directed by the sleeve, wherein the device acts in a restrictive and disabsorptive manner;

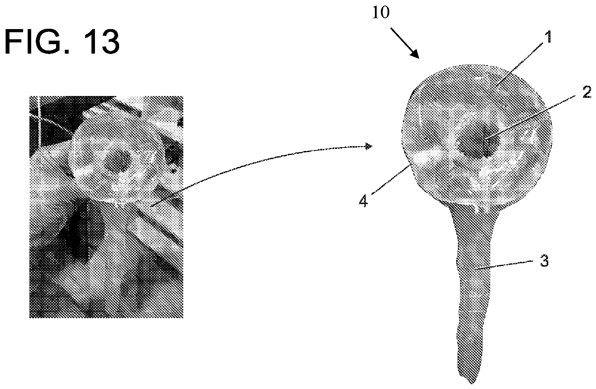

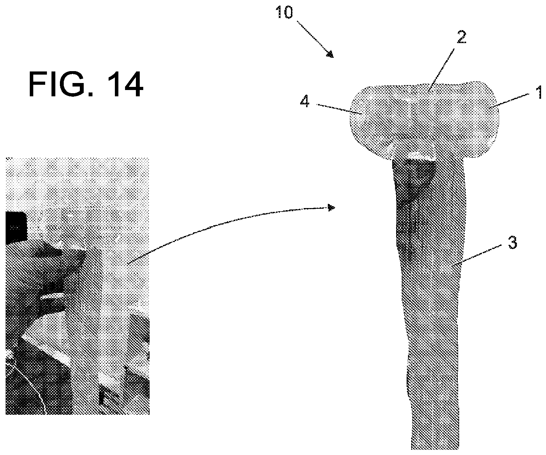

Figures 13 and 14 - show a picture and a detail of the device being held by the inventor, in a top and front view, respectively.

### Detailed description of the invention

According to the appended figures, the "GASTROINTESTINAL ENDOSCOPIC DEVICE FOR RESTRICTION AND REDUCING OF FOOD ABSORPTION, WITH A POSITIONING SYSTEM IN THE STOMACH AND EXTENSION TO THE INTESTINE", object of this invention patent application, as illustrated in figures 4, 13 and 14, is a gastrointestinal device (10) formed from an Intragastric ring (1) produced in flexible and expandable material, preferably silicone, where from its hollow central portion (2) extends a tubular sleeve (3) of the same material. Said device being designed to have its Intragastric ring (1) positioned and inflated in the stomach of a patient and extending its sleeve (3) to the intestine in order to reduce food absorption.

More specifically, the Intragastric ring (1) is presented in a retracted, deflated and compact state. The ring (1) adopts, at any place of its expandable body, an inlet valve (4) of the type with outlet blockage and whose nozzle is suitable to receive the tip of a catheter (CA).

More specifically, the tubular sleeve (3) is welded and permanently connected to the ring (1), having its body corrugated in folding ribs (5) to allow expansion and retraction. The end of the sleeve (3) connected to the ring (1) coincides with its central opening (2), from where it extends its tubular body to the opposite end, the length of which may vary depending on the application or the patient's anatomy.

The tubular sleeve (3) may be fitted with a hollow stent (6) of the spiral type, as illustrated in Figure 5, or of the rectilinear type, as illustrated in Figure 6. This stent (6) has its length extended along the length of the tubular sleeve (3) and may have its end for air, liquid or gas inlet, connected directly or not to the Intragastric ring (1), where it then receives an independent valve with catheter inlet.

Once formed the gastrointestinal device (10), it is configured to remain in the patient's stomach and intestine and resist migration, in order to both performing a weight loss treatment and combating related diseases such as type 2 diabetes.

For its positioning system, the gastrointestinal device (10) has its inlet valve (4) coupled to a catheter (CA) for gastrointestinal implant, i.e. for entering into the stomach (E) of a patient. When not fitted with a stent (6), an endoscopic forceps (EF) is inserted into the central opening (2) of the ring (1), resting on the sleeve (3). Thus, the gastrointestinal device (10) is guided inwards through the stomach of the patient. The reduced thickness, both of the sleeve (3) and the Intragastric ring (1), allows it to be entered into the stomach (E) with minimal discomfort for the patient.

As illustrated in Figure 7, the endoscopic forceps (EF) directs the tubular sleeve (3) through the Pyloric channel (P), entering the duodenum (D) and unwinding until it occupies a portion of the intestine. The Intragastric ring (1), deflated, is then positioned close to the pyloric channel (P), inside the stomach (E). Thus, when the release of fluid (liquid, air or gas) is triggered by the catheter (CA), it enters from the inlet valve (4), as illustrated in figures 9 and 10, inflating and expanding the Intragastric ring (1) above the Pyloric channel (P), thus configuring a spherical or similar-shaped balloon, which is free of tips or corners. In this way the Intragastric ring (1) remains in this position, without being able to move, when the catheter (CA) and the endoscopic forceps (EF) can then be removed.

The positioning of the tubular sleeve (3) in the duodenum and intestine may be performed before or after the intragastric ring (1) is inflated.

Alternatively, as illustrated in Figure 11, when provided with a (spiral or rectilinear) stent (6), the tubular sleeve (3) is only positioned at the entrance of the duodenum (D) when the device (10) is positioned in the stomach (E). Thus, if the stent (6) is connected directly to the valve (4) of the ring (1), it will also receive the fluid supply - liquid, air or gas, inflating and priming its body, extending and positioning the tubular sleeve (3) in the duodenum and intestine, without the need for endoscopic forceps. If the stent (6) is separated from the ring (1), a second catheter is fitted into its valve before the device (10) is entered into the stomach (E). Thus, when correctly positioned, before or after the ring (1) is inflated, the ring (1) receives its own supply of fluid to prime its body and unwind the sleeve (3) through the duodenum and part of the intestine.

In either case, the inflated Intragastric ring (1) remains stabilized immediately above the pyloric channel (P), without the need for the use of clamps or ties as in common applications.

The Intragastric ring (1) thus stabilized, as illustrated in Figure 12, allows the passage of part of the processed food (AL) in restrictive technique by occupying part of the stomach, inhibiting hunger and reducing the amount of food ingested. The device (10) provides the passage of said part of processed food through the tubular sleeve (3), stabilized along the duodenum and a portion of the intestine, which results in a disabsorptive technique, preventing absorption of the ingested food content in this part of the intestine. Therefore, by combining two techniques in a simplified construction and an easy positioning without the need for surgery, the device (10) proves to be extremely effective in the treatment against obesity and diseases associated to overweight.

The gastrointestinal device (10) is thus kept well positioned and stable, acting in a restrictive and disabsorptive manner (as occurs with a gastric bypass surgery), but without the surgical risk, in addition to being reversible or reapplicable.

## Claims

1. - "ENDOSCOPIC GASTROINTESTINAL DEVICE FOR RESTRICTION AND REDUCING OF FOOD ABSORPTION", for gastrointestinal implant in the treatment of weight reduction and combating of related diseases, such as type 2 diabetes, by combining a restrictive technique with a disabsorptive technique, wherein a gastrointestinal device (10) is formed from an Intragastric ring (1) produced in flexible and expandable material, preferably silicone, **characterized in that**, from its central hollow part (2) extending a tubular sleeve (3) of the same material, the device being designed to have its Intragastric ring (1) positioned and inflated in the stomach of a patient at the same time that it extends its sleeve (3) to the intestine in order to reduce food absorption.

2. - "ENDOSCOPIC GASTROINTESTINAL DEVICE FOR RESTRICTION AND REDUCING OF FOOD ABSORPTION", according to claim 1, **characterized in that** the Intragastric ring (1) is in a retracted, deflated and compact state, which adopts, at any place of its expandable body, an inlet valve (4) of the type with outlet blockage and whose nozzle is suitable to receive the tip of a catheter (CA).

3. - "ENDOSCOPIC GASTROINTESTINAL DEVICE FOR RESTRICTION AND REDUCING OF FOOD ABSORPTION", according to claim 1, **characterized in that** the tubular sleeve (3) is welded and permanently connected to the ring (1), having its corrugated body in folding ribs (5) to allow its expansion and retraction, wherein the end of the sleeve (3) connected to the ring (1) coincides with its central opening (2), from where it extends its tubular body to the opposite end, in a variable length according to the application or anatomy of the patient.

4. - "ENDOSCOPIC GASTROINTESTINAL DEVICE FOR RESTRICTION AND REDUCING OF FOOD ABSORPTION", according to claim 3, **characterized in that** the tubular sleeve (3) is fitted with a hollow stent (6) of the spiral or rectilinear type.

5. - "ENDOSCOPIC GASTROINTESTINAL DEVICE FOR RESTRICTION AND REDUCING OF FOOD ABSORPTION", according to claim 4, **characterized in that** the stent (6) has its length extended along the length of the tubular sleeve (3) and having its air, liquid or gas inlet end connected directly to the Intragastric ring (1) or being independent, when it receives its own valve with catheter inlet.

6. - "POSITIONING SYSTEM IN THE STOMACH AND EXTENSION TO THE INTESTINE", according to the gastrointestinal device (10) defined in claim 1, the latter being configured to remain in the stomach and intestine of the patient and resist migration, without the need for clamps or tethers, inhibiting hunger, allowing the passage of only part of the processed food (AL) and preventing the passage of this part of processed food in a portion of the intestine, and for its positioning, the device (10) has its inlet valve (4) coupled to a catheter (CA) for gastrointestinal implant, **characterized in that**, when not equipped with a stent (6), an endoscopic forceps (EF) is inserted into the central opening (2) of the ring (1), resting on the sleeve (3), the gastrointestinal device (10) being then guided inwards through the stomach (E) of the patient, where the endoscopic forceps (EF) directs the tubular sleeve (3) through the Pyloric channel (P), entering the duodenum (D) and unwinding until it occupies part of the intestine, and the Intragastric ring (1), deflated, is positioned near the Pyloric channel (P), inside the stomach (E); thus, when the release of fluid (liquid, air or gas), is triggered by the catheter (CA), it enters from the inlet valve (4), inflating and expanding the Intragastric ring (1) above the Pyloric channel (P), configuring itself as a balloon that remains in said position, without the possibility to move, when the catheter (CA) and the endoscopic forceps (EF) are then removed.

7. - "POSITIONING SYSTEM IN THE STOMACH AND EXTENSION TO THE INTESTINE", according to claim 6, **characterized in that** the positioning of the tubular sleeve (3) in the duodenum and intestine can be performed before or after the Intragastric ring (1) is inflated.

8. - "POSITIONING SYSTEM IN THE STOMACH AND EXTENSION TO THE INTESTINE", according to claim 6, **characterized in that**, when fitted with a (spiral or rectilinear) stent (6), the tubular sleeve (3) is only positioned at the entrance of the duodenum (D) when positioning the device (10) in the stomach (E), wherein the stent (6) connected directly to the valve (4) of the ring (1) also receives the fluid supply - liquid, air or gas - directed to the latter, inflating and priming its body, extending and positioning the tubular sleeve (3) in the duodenum and intestine, without the need for endoscopic forceps.

9. - "POSITIONING SYSTEM IN THE STOMACH AND EXTENSION TO THE INTESTINE" according to claim 8, **characterized in that** the stent (6) separated from the ring (1) receives its own catheter, fitted into its valve before the device (10) is inserted into the stomach (E) so that, when correctly positioned, before or after the ring (1) is inflated, it receives its own fluid supply to prime its body and unwind the sleeve (3) through the duodenum and a portion of the intestine.
